# EUROPEAN PATENT SPECIFICATION

(11) **EP 2 832 347 B1**
(45) Date of publication and mention of the grant of the patent: **31.01.2018**
(21) Application number: 14178866.1
(22) Date of filing: 29.07.2014
(51) Int. Cl.: G01N 33/50, C07K 14/72, C11B 9/00

(54) **Method for identifying patchouli odorant materials**
Verfahren zur Identifizierung von Patschuliduftstoffmaterialien
Procédé d'identification de matières odorantes de patchouli

(30) Priority: 02.08.2013 EP 13306117
(43) Date of publication of application: 04.02.2015
(73) Proprietor: Takasago International Corporation, Tokyo 144-8721 (JP)
(72) Inventor: Warr, Jonathan, 75017 Paris (FR); Fujiwhara, Mitsuhiko, Hiratsuka Kanagawa 254-0073 (JP); Emura, Makoto, Hiratsuka Kanagawa 254-0073 (JP); Lebret, Didier, Hiratsuka Kanagawa 254-0073 (JP); Lohmer, Stefan, 20091 Bresso - Milan (IT); Winnig, Marcel, 20091 Bresso - Milan (IT)
(74) Representative: Nevant, Marc

(56) References cited:
- WO-A1-02/18657
- WO-A1-2008/137993
- WO-A2-2006/002161
- WO-A2-2010/088633
- MALNIC B; HIRONO J; SATO T; BUCK LB.: "Combinatorial receptor codes for odors.", CELL, vol. 96, no. 5, 5 March 1999 (1999-03-05), pages 713-23, XP002188611,

### SEQUENCE LISTING

<110> TAKASAGO INTERNATIONAL CORPORATION
<120> METHOD FOR IDENTIFYING PATCHOULI ODORANT MATERIALS
<130> 3J248230 0132 EP OEB
<160> 3
<170> PatentIn version 3.3
<210> 1
   <211> 948
   <212> DNA
   <213> Homo sapiens
<400> 1
   atggaaattg tctccacagg aaacgaaact attactgaat ttgtcctcct tggcttctat      60
   gacatccctg aactgcattt cttgtttttt attgtattca ctgctgtcta tgtcttcatc     120
   atcataggga atatgctgat tattgtagca gtggttagct cccagaggct ccacaaaccc     180
   atgtatattt tcttggcgaa tctgtccttc ctggatattc tctacacctc cgcagtgatg     240
   ccaaaaatgc tggagggctt cctgcaagaa gcaactatct ctgtggctgg ttgcttgctc     300
   cagttcttta tcttcggctc tctagccaca gctgaatgct tactgctggc tgtcatggca     360
   tatgaccgct acctggcaat ttgctaccca ctccactacc cactcctgat ggggcccaga     420
   cggtacatgg ggctggtggt cacaacctgg ctctctggat ttgtggtaga tggactggtt     480
   gtggccctgg tggcccagct gaggttctgt ggccccaacc acattgacca gttttactgt     540
   gactttatgc ttttcgtggg cctggcttgc tcggatccca gagtggctca ggtgacaact     600
   ctcattctgt ctgtgttctg cctcactatt ccttttggac tgattctgac atcttatgcc     660
   agaattgtgg tggcagtgct gagagttcct gctggggcaa gcaggagaag ggctttctcc     720
   acatgctcct cccacctagc tgtagtgacc acattctatg gaacgctcat gatcttttat     780
   gttgcaccct ctgctgtcca ttcccagctc ctctccaagg tcttctccct gctctacact     840
   gtggtcaccc ctctcttcaa tcctgtgatc tataccatga ggaacaagga ggtgcatcag     900
   gcacttcgga agattctctg tatcaaacaa actgaaacac ttgattga                  948
<210> 2
   <211> 315
   <212> PRT
   <213> Homo sapiens
<400> 2
   Met Glu Ile Val Ser Thr Gly Asn Glu Thr Ile Thr Glu Phe Val Leu
   1               5                   10                  15
   Leu Gly Phe Tyr Asp Ile Pro Glu Leu His Phe Leu Phe Phe Ile Val
               20                  25                  30
   Phe Thr Ala Val Tyr Val Phe Ile Ile Ile Gly Asn Met Leu Ile Ile
           35                  40                  45
   Val Ala Val Val Ser Ser Gln Arg Leu His Lys Pro Met Tyr Ile Phe
       50                  55                  60
   Leu Ala Asn Leu Ser Phe Leu Asp Ile Leu Tyr Thr Ser Ala Val Met
   65                  70                  75                  80
   Pro Lys Met Leu Glu Gly Phe Leu Gln Glu Ala Thr Ile Ser Val Ala
                   85                  90                  95
   Gly Cys Leu Leu Gln Phe Phe Ile Phe Gly Ser Leu Ala Thr Ala Glu
               100                 105                 110
   Cys Leu Leu Leu Ala Val Met Ala Tyr Asp Arg Tyr Leu Ala Ile Cys
           115                 120                 125
   Tyr Pro Leu His Tyr Pro Leu Leu Met Gly Pro Arg Arg Tyr Met Gly
       130                 135                 140
   Leu Val Val Thr Thr Trp Leu Ser Gly Phe Val Val Asp Gly Leu Val
   145                 150                 155                 160
   Val Ala Leu Val Ala Gln Leu Arg Phe Cys Gly Pro Asn His Ile Asp
                   165                 170                 175
   Gln Phe Tyr Cys Asp Phe Met Leu Phe Val Gly Leu Ala Cys Ser Asp
               180                 185                 190
   Pro Arg Val Ala Gln Val Thr Thr Leu Ile Leu Ser Val Phe Cys Leu
           195                 200                 205
   Thr Ile Pro Phe Gly Leu Ile Leu Thr Ser Tyr Ala Arg Ile Val Val
       210                 215                 220
   Ala Val Leu Arg Val Pro Ala Gly Ala Ser Arg Arg Arg Ala Phe Ser
   225                 230                 235                 240
   Thr Cys Ser Ser His Leu Ala Val Val Thr Thr Phe Tyr Gly Thr Leu
                   245                 250                 255
   Met Ile Phe Tyr Val Ala Pro Ser Ala Val His Ser Gln Leu Leu Ser
               260                 265                 270
   Lys Val Phe Ser Leu Leu Tyr Thr Val Val Thr Pro Leu Phe Asn Pro
           275                 280                 285
   Val Ile Tyr Thr Met Arg Asn Lys Glu Val His Gln Ala Leu Arg Lys
       290                 295                 300
   Ile Leu Cys Ile Lys Gln Thr Glu Thr Leu Asp
   305                 310                 315
<210> 3
   <211> 969
   <212> DNA
   <213> Homo sapiens
<400> 3
   atggagcccc aaaatacctc cactgtgact aactttcagc tgttaggatt ccagaacctt     60
   cttgaatggc aggccctgct ctttgtcatt ttcctgctca tctactgcct gaccattata    120
   gggaatgttg tcatcatcac cgtggtgagc cagggcctgc gactgcactc ccctatgtac    180
   atgttcctcc agcatctctc ctttctggag gtctggtaca cgtccaccac tgtgcccctt    240
   ctcctagcca acctgctgtc ctggggccaa gccatctcct tctctgcctg catggcacag    300
   ctctacttct tcgtattcct cggcgccacc gagtgctttc tcctggcctt catggcctat    360
   gaccgttacc tggccatctg cagcccactc cgctacccct tcctcatgca tcgtgggctc    420
   tgtgccaggt tggtggtggt ctcctggtgc acaggggtca gcacaggctt tctgccttcc    480
   ctgatgattt ccaggttgga cttctgtggg cgcaatcaga ttaaccattt cttctgcgac    540
   ctcccgccac tcatgcagct ctcctgttcc agagtttata tcaccgaggt gaccatcttc    600
   atcctgtcaa ttgccgtgct gtgcatttgt ttttttctga cactggggcc ctatgttttc    660
   attgtgtcct ccatattgag aatcccttcc acctctggcc ggagaaagac cttttccaca    720
   tgtggctccc acctggctgt tgtcactctc tactacggga ccatgatctc catgtatgtg    780
   tgtcccagtc cccacctgtt gcctgaaatc aacaagatca tttctgtctt ctacactgtg    840
   gtcacaccac tgctgaaccc agttatctac agcttgagga acaaagactt caaagaagct    900
   gttagaaagg tcatgagaag gaaatgtggt attctatgga gtacaagtaa aaggaagttc    960
   ctttattga                                                            969

## Description

### Technical Field of the Invention

The invention relates to the identification of Patchouli odorant materials by a method comprising detecting the activity of the olfactive receptor OR11A1 (Olfactory Receptor, family 11, subfamily A, member 1).

### Background of the Invention

Humans perceive an immense variety of chemicals as having distinct odors. Odor perception initiates in the nose, where odorants are detected by a large family of olfactory receptors (ORs).

The olfactory receptor proteins are members of a large family of G-protein-coupled receptors (GPCRs) arising from single coding-exon genes. Olfactory receptors share a 7-transmembrane domain structure with many neurotransmitter and hormone receptors and are responsible for the recognition and G protein-mediated transduction of odorant signals.

Volatile compounds of different natural or artificial origins are detected by a specific set of G-protein coupled receptors located in the respiratory epithelium. It has been proposed that one odorant is capable of activating multiple olfactory receptors (ORs) and that one OR is capable of detecting different odorants (Malnic et al., 1999). The human genome contains ∼400 intact olfactory receptor genes belonging to two main classes. Class I, "fish-like receptors" ORs presumably detect water soluble odorants, whereas class II "tetrapod specific receptors" ORs are supposed to respond to airborne volatiles (Glusman et al., 2000).

OR11A1 (Olfactory Receptor, family 11, subfamily A, member 1), also known as hs6M1-18, AC004174-A, dJ994E9.6, OR6-30, HsOR6.3.21 or ORL524, belongs to a small subset of 8 structurally related receptors (**http://genome.weizmann.ac.il/horde**). Recently, Adipietro et al. demonstrated activation of OR11A1 by 2-ethylfenchol (Adipietro et al., PLoS Genet. 2012).

WO 2008/088633 describes *inter alia* a method for identifying a second test compound that mimics the odor of a first test compound or composition, the method comprising a) contacting a panel of odorant receptors with the second test compound, b) testing the effect of the second test compound on the activity in a functional assay of at least two odorant receptors in the panel, and c) comparing the odorant activity profile of the second test compound obtained in b) with the odorant activity profile of the first test compound or composition.

Patchouli oil is produced by steam distillation of the dried leaves of *Pogostemon cablin* (Blanco) Benth. (*Lamiaceae*). It is a reddish-brown to greenish-brown, more or less viscous liquid with a characteristic, slightly camphoraceous, woody balsamic odor. Although the sesquiterpene alcohol (-)-patchoulol [CAS N° 5986-55-0] is the main component of patchouli oil (27 - 35%), this compound contributes less to the characteristic odor of the oil than norpatchoulenol [CAS N° 41429-52-1] present only at a concentration of 0.35 - 1%. Other typical constituents are (+)-α-bulnesene [CAS N° 3691-11-0] (13- 21 %), (-)-α-guajene [CAS N° 3691-12-1] (11 - 16 %), (-)-β-patchoulene [CAS N° 514-51-2] (1.8 - 3.5 %), and (-)-seychellene [CAS N° 20085-93-2] (1-3%) (see 'Common Fragrance and Flavor Materials, 5th Ed. Horst Surburg and Johannes Panten Copyright 2006 WILEY-VCH Verlag GmbH & Co. KGaA, Weinheim ISBN: 3-527-31315-X, pp 223-224).

Patchouli is an odor note well-known to perfumers and has a distinctive well-balanced odor with camphoraceous, earthy, and woody sides, accompanied by balsamic and floral facets. Patchouli oil, or constituents thereof, has (have) been and is (are) still used in many fragrances (Kraft in Chemistry & Biodiversity - Vol. 5 (2008), pp970-999, Verlag Helvetica Chimica Acta AG, Zürich). Patchouli oil is notably key to the Chypre accord, in which it is combined with a fresh citrus top note around bergamot oil, a floral heart of rose and jasmin odorants, and a musky oakmoss fond (Kraft, op. cit.).

However, as of today, there are no satisfactory synthetic substituents for patchouli that are available to the perfumer because of cost and supply reasons. Given in particular the cost of good of patchouli oil, it would therefore be advantageous to have such synthetic substituents at hand.

### Summary of the Invention

In one aspect, the present invention accordingly provides a method for identifying a candidate patchouli odorant material, comprising the steps of:
1) contacting a sample expressing the OR11A1 receptor with a test compound, and
2) measuring the activity of the OR11A1 receptor as defined in claim 1.

In another aspect, the present invention is related to a method for identifying a patchouli odorant material, comprising the steps of:
a) identifying a candidate patchouli odorant material by the method as described above, and
b1) subjecting the identified candidate patchouli odorant material to a long-lasting test, wherein the candidate passes the test if it retains at least 10% of its initial concentration after 1 hour.

In a further aspect, the present invention relates to a method for identifying a patchouli odorant material, comprising the steps of:
a) identifying a candidate patchouli odorant material by the method described above, and
b2) subjecting the identified candidate to an olfactive test as defined in claim 11.

In yet a further aspect, the present invention relates to the use of a sample expressing the OR11A1 receptor for identifying a candidate patchouli odorant material upon enhancement of the activity of said receptor by said material.

### Detailed Description of the Invention

In a first aspect, the present invention provides a method for identifying a candidate patchouli odorant material, comprising the steps of or consisting in:
1) contacting a sample expressing the OR11A1 receptor with a test compound, and
2) measuring the activity of the OR11A1 receptor as defined in claim 1.

The present invention is based on the surprising finding that the olfactory receptor OR11A1 is specifically responsive to patchouli oil. As explained in detail in the examples, 65 olfactory receptors were tested for their response to patchouli oil. As a result of the tests, only OR11A1 was found to be responsive to patchouli oil. Accordingly, the OR11A1 receptor is a useful tool to screen for patchouli odorant materials.

As used herein, the term "patchouli odorant material" means an individual ingredient, agent or compound or mixtures thereof having a well-characterized patchouli odor note and which thus can be used for replacing, partially or totally, patchouli oil in a composition. As explained above, the patchouli oil odor is a well-known and characteristic odor that perfumers can easily identify. Patchouli oil is also noted for its remenance, so it is frequently used in fabric softener fragrances for example, to leave a lasting scent on dry clothes after washing.

The method of the invention comprises a first step (step 1)) where a sample expressing the OR11A1 receptor is contacted with the test compound.

As used herein, the term "expressing the OR11A1 receptor" refers to the expression of a functional olfactory receptor OR11A1. Specifically, the sample expresses the OR11A1 receptor which can be activated, namely transduce the downstream signalling, following the interaction with the corresponding odorant receptor ligand.

In one embodiment, the expression of OR11A1 is localized to the cell membrane of the sample. Consequently, the sample may for example also express at least one auxiliary protein important for efficient receptor trafficking to the plasma membrane. Also, the sample can further express a stimulatory G-protein (also called Gαs), preferably a Gαolf G-protein.

In a preferred embodiment, the sample to be used in the method of the present invention comprises a cell, and in particular a mammalian cell.

In preferred embodiments, the sample is a cell line expressing the OR11A1 receptor or membranes preparations of the cells of said cell line. Advantageously, the sample expressing the OR11A1 receptor is a cell line selected from the group consisting of HEK293T, CHO (Chinese hamster ovary), HeLa and membranes preparations of said cells. In a further embodiment, expression of the OR11A1 receptor is provided by using the nucleotide or amino acid sequences corresponding to the OR11A1 receptor.

In one embodiment, the cDNA of OR11A1, preferably the cDNA corresponding to human OR11A1 of sequence SEQ ID NO: 1, is used:

In a preferred embodiment, a nucleotide sequence is used which is a nucleotide sequence encoding the human OR11A1 receptor (notably SEQ ID NO: 1) or any nucleotide sequence encoding either the polypeptide of SEQ ID NO: 2 (see below) or a polypeptide having an amino acid sequence identity of at least 85 %, preferably at least 90 % and more preferably at least 95 %, with SEQ ID NO: 2.
SEQ ID NO: 2

As used herein, the term "sequence identity" means the identity of sequence between two amino acid sequences over the total length of the sequences when optimally aligned, such as by using the program ClustalW (http://www.clustal.org/; Thompson JD, Higgins DG, Gibson TJ. (1994); Acids Res., 22, 4673-4680). (Gap opening penalty = 10; Gap extension penalty = 0.1).

By way of information, the sequence identity (%) of receptor OR11A1 with various odorant receptors as disclosed in WO 2006/002161 and WO 2008/137993 is shown in the table below.

| | MOR23-1 | OR7D4 | S50 | S6 | MOR31-4 | MOR31-6 | S18 | MOR32-11 | MOR32-5 | S46 |
|---|---|---|---|---|---|---|---|---|---|---|
| OR11A1 | 28 | 37 | 35 | 34 | 28 | 28 | 29 | 30 | 31 | 28 |

In some embodiments, the nucleotide sequence may be included in a construct within any one of a variety of expression vectors, as long as it is able to replicate and express the construct in a host, leading to the expression of the corresponding polypeptide. The possible constructs are also not limited provided the sequence is operably linked to an appropriate expression control sequence(s) and any other requirements to direct the expression of the OR11A1 receptor.

In preferred embodiments, the sequence of the OR11A1 receptor is directly tagged or indirectly associated with a reporting system leading to a readable or measureable signal once the OR11A1 receptor is activated by ligand(s), so that the signal transduction following activation of the receptor can be detected, measured and/or monitored.

Any type of reporting system can be used in the method of the present invention. For example, the sample may also express a reporting system comprising a gene whose transcription is driven by a response element whose activity is an indication of OR11A1 receptor activation, preferably a cAMP response element which is stimulated by adenylate cyclase which is activated by the stimulatory G-protein (Gαs), such as Gαolf G-protein. Any method for expressing the OR11A1 receptor can be used, and preferably transfection of the expression vector containing the constructs comprising the sequence encoding the OR11A1 receptor.

The term "test compound" refers to any chemical entity that can be used in a composition as odorant, and especially in the perfumery or cosmetic field in general. Test compounds comprise both known and potential odorant compounds.

In one embodiment, the method of the present invention is implemented with a test compound having a molecular weight of greater than 183, more preferably equal to or greater than 200. Preferably, the test compound has a molecular weight of 300 or less, more preferably of 275 or less. Even more preferably, the method of the present invention is implemented with a test compound having a molecular weight in the range from 200 to 275.

In one embodiment, the test compound is not 2-ethylfenchol (CAS N° 18368-91-7).

In one embodiment, the step of contacting the sample with the test compound is carried out using different concentrations of the test compound to determine the effective amount of said test compound which is able to activate the OR11A1 receptor.

In a second step (step 2)), the activity of the OR11A1 receptor is measured. The test compound is considered as a candidate patchouli odorant material if it enhances the activity of said OR11A1 receptor.

The step of measuring the activity of the OR11A1 receptor comprises the steps of or consists in:
i) measuring the response of the OR11A1 receptor to the test compound and the response of the OR11A1 receptor to the assay buffer (baseline), and calculating a first fold of change (FOC1: response OR11A1 to test compound/response OR11A1 to baseline);
ii) measuring the response of a control sample which does not express the OR11A1 receptor to the test compound and the response of the control sample which does not express the OR11A1 receptor to the assay buffer (baseline), and calculating a second fold of change (FOC2: response control sample to test compound/response control sample to baseline);
wherein enhancement of the activity of the OR11A1 receptor occurs if the ratio of FOC1 to FOC2 is higher than 1.

In one embodiment, enhancement of the activity of the OR11A1 receptor occurs if FOC1 is higher than FOC2, and if the difference between FOC1 and FOC2 is statistically significant with p<0.01, as determined e.g. by Student's *t*-Test.

For example, a control sample used in step ii) above may be a sample which express a non-functional OR11A1 receptor or an empty expression vector corresponding to the host vector used for expressing OR11A1 receptor in the sample (control vector) in step 1) above.

In a preferred embodiment, enhancement of the activity of the OR11A1 receptor occurs if the ratio of FOC1 to FOC2 is higher than 1 and FOC1 is of at least 1.5, preferably and FOC1 is of at least 1.8, more preferably and FOC1 is of at least 2.

Any method known in the art may be used to read or measure the response of the OR11A1 receptor when the sample is contacted with the test compound. The specific way to measure/read the response and thus determine the presence or absence of enhancement of the activity of the OR11A1 receptor generated by the test compound is adapted with relation to the readable or measurable signalling system which has been selected in the sample expressing the OR11A1 receptor.

According to a preferred embodiment, the activity of the OR11A1 receptor is detected by using a reporting system leading to a readable or measureable signal if the test compound elicits a response of the OR11A1 receptor . As mentioned above, and in a further embodiment, the reporting system to be used may be such as, but not limited to, an imaging system, a fluorescence system, an enzymatic system, a binding system, etc. For example, the following reporter systems may be used in a further preferred embodiment: calcium imaging with fluorescent dyes, photoproteins such as GFP, aequorin, enzymatic reporter system such as luciferase, chloramphenicol acetyltransferase, β-galactosidase, alkaline phosphatase, horse radish peroxidase, GTPgammaS binding test, measurement of changes in cAMP levels, and the like. Advantageously, a reporting system comprising a gene whose transcription is driven by a response element whose activity is an indication of OR11A1 receptor modulation may be used, preferably the gene transcription is driven by a cAMP response element which is stimulated by adenylate cyclase which is activated by the stimulatory G-protein (Gαs), such as Gαolf G-protein.

The method described above makes it possible to identify patchouli odorant materials that potentially allow at least partial substitution, preferably total substitution, of patchouli oil or patchouli alcohol, in particular in perfumery in a fragrance composition.

Thus in another aspect, the present invention relates to a method for identifying a patchouli odorant material that can be substituted for patchouli oil or any other constituent(s) contained in patchouli oil contributing to the patchouli characteristic odor, which method comprises the steps of or consists in:
a) identifying a candidate patchouli odorant material by the method described above; and
b1) subjecting the identified candidate to a long-lasting test;
wherein the candidate material passes the test if it retains at least 10% of its initial concentration after 1 hour.

Any method for determining if a material will remain during time when applied to a specified/selected substrate may be used for the long-lasting test. The substrate can for example be skin, fabric, hair etc; the substrate can also be a scent strip. Therefore any method which measures the residual quantity/concentration after a certain time can be used for measuring the long-lasting property of patchouli odorant materials according to the present invention. An example of an easy and reproducible method is to measure the quantity/concentration of the material on a substrate over time by gas chromatography, such as GC-MS (gas chromatography/mass spectrometry).

In one embodiment, the successful candidate retains at least 20 %, preferably at least 30 %, preferably at least 40 %, preferably at least 50 %, or preferably at least 60 %, of its initial concentration after 1 hour, as measured as mentioned above.

In one embodiment, the successful candidate retains at least 30 % of its initial concentration after 2 hours, more preferably at least 20 % of its initial concentration after 4 hours, and most preferably at least 10 % of its initial concentration after 8 hours, as measured as mentioned above.

In one embodiment, the long-lasting analysis is carried out as follows: 50 µl of a 1 % solution of the identified candidate to be tested in alcohol, preferably ethanol, are applied on a scent strip, preferably a cellulosic scent strip, to be dosed. The strip is allowed to dry under ambient laboratory conditions, with normal room air movement, in particular no fan, heating or any mean or device to accelerate evaporation. After different times, each strip or at least the part of the scent strip including the area where the solution of the identified candidate to be tested was applied is extracted using 3 ml of alcohol, preferably ethanol, containing an internal standard, such as methyl decanoate. The extracts are then analyzed by gas chromatography and the result is normalized for the principal molecular ion of the identified candidate using the value for the internal standard.

For example, the extracts are analysed by GC-MS according to any conventional analysis performed by those skilled in GC-MS measurements, or any standard methods provided by the instrument supplier. This analysis can be conducted by using a polar or non-polar gas chromatography column, and preferably a non-polar gas chromatography column. As example of non-polar gas chromatography columns, a column BC-WAX (50m * 0.25mm * 0.15µm), which is a GC Sciences column supplied by Interchim (Montluçon, France), and a column HP-5MS UI (30m * 0.25mm * 0.25µm), which is an Agilent column supplied by Chromoptic (Courtaboeuf, France), can be mentioned.

In another aspect, the identification of a patchouli odorant material suitable as a patchouli oil substitute is carried out by a method comprising the steps of or consisting in:
a) identifying a candidate patchouli odorant material by the method described above; and
b2) subjecting the identified candidate to an olfactive test.

The olfactive test comprises comparing a sample containing patchouli oil, preferably a sample containing only patchouli oil, with a sample containing patchouli oil and the identified candidate, preferably a sample containing patchouli oil, the identified candidate and a solvent. The identified candidate passes the test if there is no perceptible sensory difference between the two samples, this difference being assessed by methods well known to those skilled in the art of perfumery. In one embodiment the olfactive test is carried out following the standard test method for sensory analysis (triangle test) as defined in standard ASTM (American Society for Testing and Materials ) E 1885 - 97 (edition approved July 10, 1997; published August 1998). This test method covers a procedure for determining whether a perceptible sensory difference exists between samples of two products. In the protocol of the olfactive test, a comparison is made between two samples only, namely a sample containing 100 wt% patchouli oil and a sample containing 50 wt% patchouli oil + x wt% "candidate" + (100-50-x) wt % solvent (typically dipropylene glycol, DPG). A candidate material passes the test if there is no perceptible sensory difference between the two samples in the triangle test as defined in the above-mentioned standard. The candidate material passing the olfactive test is thus suitable as substituent for the patchouli characteristic odor, and can be formulated into a fragrance composition. The skilled person will appreciate that the amount (x wt%) of candidate material to be used depends on the olfactive strength of that material.

In another aspect, the identification of a patchouli odorant material suitable as a patchouli oil substitute is carried out by a method comprising the steps of or consisting in:
a) identifying a candidate patchouli odorant material by the method described above;
b1) subjecting the identified candidate to a long-lasting test;
b2) subjecting the identified candidate to an olfactive test;
said long-lasting test and olfactive test being as defined above.

In one embodiment, the steps of this method are carried out in the following order: a), b1), b2).

In another embodiment, the steps of this method are carried out in the following order: a), b2), b1).

In yet a further aspect, the present invention is related to the use of a sample expressing OR11A1 receptor for identifying a candidate patchouli odorant material upon enhancement of the activity of said receptor by said material. All the preferred embodiments mentioned above regarding the method of identifying a patchouli odorant material are also preferred embodiments of the use according to the present invention.

The invention is illustrated by the following, non-limiting examples and the annexed drawings, wherein:
- **Figures 1A** **and** **1B** show the responses of each of the 65 tested olfactory receptors towards 10µM concentration of controls isoproterenol or androstenone (A) and 10µM, 30µM and 100µM concentrations of patchouli oil (B).
- **Figure 2** represents a dendrogram of the OR11A1 subfamily.
- **Figures 3A to 3C** show the concentration-dependent responses of OR11A1 and OR11L1 after stimulation with patchouli oil (A) and R2335 (B) or the direct comparison of responses of OR11A1 towards patchouli oil and R2335 (C).
- **Figure 4** shows the responses of OR11A1 related receptors towards the odorants (break in y-axis at 2) patchouli alcohol and patcho 315.
- **Figure 5** shows the responses of OR11A1 towards the odorants patcho 315, patchouli alcohol, terranol, andrane, pogostone and norpatchouli alcohol.
- **Figure 6** shows the concentration-dependent responses of OR11A1 after stimulation with the odorants pogostone, norpatchouli alcohol, patchouli alcohol and patcho 315.
- **Figures 7A and 7B** show the long-lasting analysis of patchouli alcohol.
- **Figure 8** shows the long-lasting analysis of pogostone.

### EXAMPLES

### Cell lines, Medium, and Cell Culture Conditions

The experiments were performed using a HEK293 cell line (deposited at the ATCC under accession n° CRL-1573) stably expressing the Gαolf G-protein and the cyclic nucleotide gated ion channel A2 subunit (CNGA2) (herein called HEKOlf). As described by Shirokova et al., 2005, the human Gαolf cDNA was cloned into the mammalian expression vector pPuro and cDNA for the bovine cyclic nucleotide gated ion channel A2 (CNGA2) subunit was cloned into the mammalian expression vector pcDNA3. Both constructs were transfected using Lipofectamine2000 (Invitrogen) following the manufacturer's instructions. Cellular clones stably expressing Gαolf and CNGA2 were obtained by antibiotic selection with 0.5µg/ml Puromycin and 200µg/ml Geneticin.

The cell line HEKOlf was maintained in Dulbecco's modified Eagle Medium (DMEM, Euroclone) supplemented with 10% FBS (Fetal Bovine Serum, Euroclone cat. ECS0180L), 1% Penicillin/Streptomycin (Euroclone cat. B3001D), 50 µg/ml G418, 25 µg/ml Hygromycin, and 2.5 µg/ml Blasticidin, 0.5 µg/ml Puromycin, and 3 µg/ml Zeocin. Standard propagation conditions consist of seeding 1.5 - 1.8x10⁶ cells in a T75 flask twice a week, recovering about 10-15x10⁶ cells at ∼ 80% confluence.

### Cloning of Human Olfactory Receptors

The cDNA of the human olfactory receptors was obtained by PCR from human genomic DNA (Clonetech), and cloned in frame 3' from a transmembrane localization epitope into a pcDNA5 expression vector (Bufe et al., 2002). The correct identity of every single olfactory receptor was verified by automated sequencing.

The correspondence between the names of the olfactory receptors as used herein and in the figures and the respective GeneBank accession numbers are described in Table 1 below:

**Table 1**

| **Receptor** | **GeneBank Accessions** | **Name** |
|---|---|---|
| OR6A2 | NM_003696 | OR1 |
| OR6Q1 | NM_001005186 | OR2 |
| OR6C68 | NM_001005519 | OR3 |
| OR6F1 | NM_001005286 | OR4 |
| OR6X1 | NM_001005188 | OR5 |
| OR6N2 | NM_001005278 | OR6 |
| OR7A10 | NM_001005190 | OR7 |
| OR7E24 | BK004227 | OR8 |
| OR7D4 | NM_001005191.2 | OR9 |
| OR8K5 | NM_001004058 | OR10 |
| OR8A1 | NM_001005194 | OR11 |
| OR8B4 | NM_001005196 | OR12 |
| OR9G1 | NM_001005213 | OR13 |
| OR9K2 | NM_001005243 | OR14 |
| OR10A5 | NM_178168 | OR15 |
| OR10V1 | NM_001005324 | OR16 |
| OR10G2 | NM_001005466 | OR17 |
| OR10G8 | NM_001004464 | OR18 |
| OR11A1 | BK004208.1 | OR19 |
| OR11L1 | NM_001001959.1 | OR20 |
| OR12D2 | NM_013936 | OR21 |
| OR12D3 | NM_030959 | OR22 |
| OR13C2 | NM_001004481 | OR23 |
| OR13C3 | NM_001001961 | OR24 |
| OR13C4 | NM_001001919 | OR25 |
| OR13C5 | NM_001004482 | OR26 |
| OR13C8 | NM_001004483 | OR27 |
| OR13C9 | NM_001001956 | OR28 |
| OR14A16 | NM_001001966 | OR29 |
| OR14I1 | NM_001004734 | OR30 |
| OR1A1 | NM_014565 | OR31 |
| OR1D2 | NM_002548 | OR32 |
| OR1L1 | NM_001005236 | OR33 |
| OR1Q1 | NM_012364 | OR34 |
| OR2A1 | NM_001005287 | OR35 |
| OR2A7 | NM_001005328 | OR36 |
| OR2B11 | NM_001004492 | OR37 |
| OR2F2 | NM_001004685 | OR38 |
| OR2J3 | NM_001005216 | OR39 |
| OR2AE1 | NM_001005276 | OR40 |
| OR2Z1 | NM_001004699 | OR41 |
| OR2T1 | NM_030904 | OR42 |
| OR2T34 | NM_001001821 | OR43 |
| OR2AJ1 | AB065626 | OR44 |
| OR2AG2 | NM_001004490 | OR45 |
| OR2AP1 | AB065868 | OR46 |
| OR2AT4 | NM_001005285 | OR47 |
| OR2S2 | NM_019897 | OR48 |
| OR3A1 | NM_002550 | OR49 |
| OR3A4 | NM_001005334 | OR50 |
| OR4A15 | NM_001005275 | OR51 |
| OR4S1 | NM_001004725 | OR52 |
| OR4D1 | NM_012374 | OR53 |
| OR4Q3 | NM_172194 | OR54 |
| OR4F15 | NM_001001674 | OR55 |
| OR4E2 | NM_001001912 | OR56 |
| OR5AP2 | NM_001002925 | OR57 |
| OR5I1 | NM_006637 | OR58 |
| OR5AC2 | NM_054106 | OR59 |
| OR5K4 | NM_001005517 | OR60 |
| OR5A1 | NM_001004728 | OR61 |
| OR5AK2 | NM_001005323 | OR62 |
| OR5D13 | NM_001001967 | OR63 |
| OR5J2 | NM_001005492 | OR64 |
| OR5M1 | NM_001004740 | OR65 |
| | | |
| pcDNA5 | | empty vector |
| OR7D4 | NM_001005191 | positive control receptor |
| hTAS2R16 | NM_016945 | negative control receptor |

### Ligands and Buffers

All odorants were stored in the dark at 4°C upon delivery. They were prepared at 100mM stock solutions using DMSO as a solvent and stored at -20°C. On the day of the experiment the different stock solutions were directly diluted in the assay buffer (Tyrode's buffer) having the following composition: 5mM KCI, 130mM NaCl, 2mM CaCl₂, 5mM NaHCO₃, 1mM MgCl₂, 20mM HEPES, pH7.4. The odorants were used in concentrations indicated in tables 2-4 below.

### Instrumentation and Disposables

The experiments were performed using FLIPR^{TETRA} (Molecular Devices). The cells were transfected and seeded in black 384 well polystyrene assay plates, black/clear bottom (MATRIX Part # CPL-4332).

### Transient Transfection with Lipofectamine 2000

All transient transfections were performed with Lipofectamine 2000 (Invitrogen) according to the manufacturer's protocol. 10µl Lipofectamine 2000 were diluted in 500 µl DMEM and incubated for 5 minutes at room temperature. At the same time, 3µg of plasmid DNA were diluted in 500µl DMEM and added to the Lipofectamine 2000 mix to obtain a final volume of 1000µl. After incubation for a further 30 minutes at room temperature, the DNA-Lipofectamine complex was added to 1000µl of a cell suspension containing 1.600.000 cells/ml. Subsequently, 25µl per well of the complete mixture were seeded in black 384 well polystyrene assay plates. 25µl DMEM containing 20% FBS were added to each well 3 hours post-transfection.

### Luminescence Measurement at FLIPR^{TETRA}

The cDNAs for all olfactory receptors and an empty control vector were co-transfected with pGL4.29 (Promega). The pGL4.29 vector contains a cyclic adenosine monophosphate (cAMP) response element (CRE) that drives the transcription of the luciferase reporter gene *luc2P (Photinus pyralis). luc2P* is a synthetically-derived luciferase sequence with humanized codon optimization that is designed for high expression and reduced anomalous transcription.

In this assay, the interaction of an odorant with its cognate olfactory receptor leads to the activation of the human Gαolf G-protein. Gαolf is a stimulatory G-protein (Gαs) and activates adenylate cyclase, which converts adenosine triphosphate (ATP) into cAMP. Therefore, the activity of the transcribed luciferase can be used as an indication of olfactory receptor activation. 24 hours post-transfection the medium was removed from the transfected cells and 30 µl Tyrode's buffer containing the diluted odorants were added and incubated for 6 hours at 37°C. The cells were then transferred into an automated fluorometric imaging plate reader (FLIPR^{TETRA}, Molecular Devices). Subsequently, 25µl of a mixture composed of Triton X-100 and luciferin (1:1) were added. The addition of this mixture led to cell lyses and allowed interaction of the luciferase with its substrate luciferin resulting in a detectable emission of light.

### Data Analysis

The data obtained from different well replicates were analyzed with Excel, ScreenWorks, and SigmaPlot 10. The responses of each receptor to the odorants were divided by the mean responses of the receptor towards Tyrode's buffer (fold of change FOC1). The specificity of FOC1 was checked by comparing with a second FOC (FOC2) obtained by stimulating a control sample, consisting in an empty expression vector, with the same odorants and Tyrode's buffer. The mean and standard deviation of the resulting fold of change values were calculated and plotted in vertical bar charts or concentration-response curves fitted by the SigmaPlot 10 software.

### RESULTS

### Example 1: Identification of human OR for patchouli odorant

To identify possible interactions of patchouli essential oil with different human olfactory receptors, HEKOlf cells were transiently transfected with plasmid DNA for 65 human olfactory receptors and, separately, with a control plasmid that served as a negative control, as described above.

Androstenone was used as an activator of OR7D4, which served as a positive control for general olfactory receptor activity (Keller et al., 2007). Isoproterenol activates the beta-2-adrenergic receptor that is endogenously expressed and coupled to human Gαolf in HEKOlf cells. Cellular activity elicited by isoproterenol proofed the viability of the cells and the correct functionality of the signalling cascade.

The different odorant tested on the human olfactory receptors are as mentioned in Table 2:

**Table 2**

| **Compound** | **Concentrations used (µM)** | Provider |
|---|---|---|
| androstenone | 10 | Sigma-Aldrich |
| isoproterenol | 10 | Sigma-Aldrich |
| patchouli oil | 0.1/0.3/1/3/10/30/60/100 | Takasago |

Each compound plate contained only one odorant to be tested and was applied onto the three differently transfected plates containing all 65 available olfactory receptors. In addition, OR7D4 (positive control) and an empty pcDNA5 expression vector (mock, negative control) were present on each plate. The human bitter taste receptor TAS2R16 (Bufe et al., 2002) served as an additional negative control.

The application of 10 µM isoproterenol led to detectable signals in all transfected cells, thus proofing cell vitality and the correct functionality of the signalling cascade. In addition, 10 µM androstenone robustly activated OR7D4, whereas all the 65 olfactory receptors tested, including the negative control receptor (hTAS2R16), did not respond to this compound.

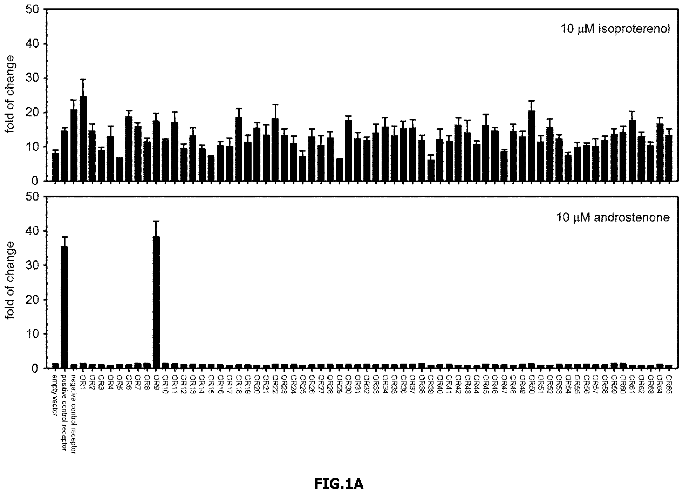

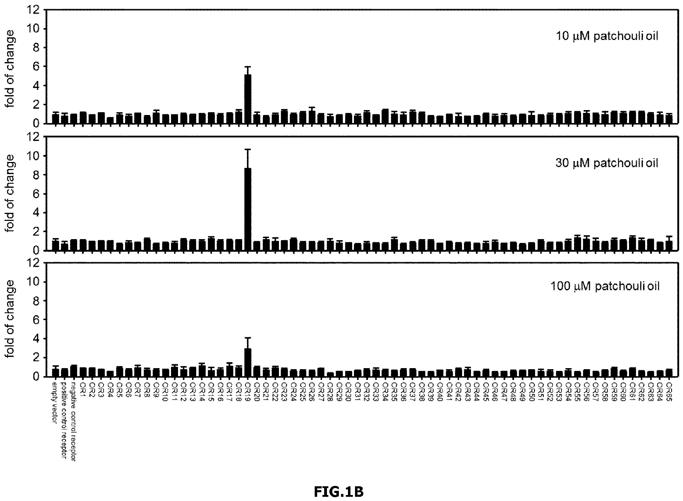

The screening of the odorants against the set of 65 human olfactory receptors (OR) identified OR11A1 to be specifically responsive to patchouli oil. No other human OR responded to this odorant. Moreover, all tested concentrations activated this receptor. Figures 1A and 1B show the responses of the tested OR to isoproterenol, androstenone and to three concentrations of patchouli essential oil.

### Example 2: Characterization of the identified Human olfactory Receptor for Patchouli Oil

To determine the potency and efficacy of patchouli oil, HEKOlf cells were transfected with the cDNA for OR11A1 (SEQ ID NO: 1). Additionally, cells were also transfected with the cDNA of OR11L1 of sequence SEQ ID NO: 3, a human olfactory receptor that belongs to the same subfamily as OR11A1.
SEQ ID NO: 3

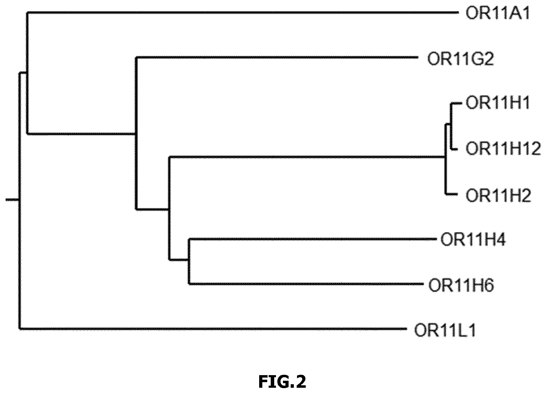

Figure 2 represents a dendrogram of the OR11A1 subfamily.

Cells transfected with OR11A1 and OR11L1 cDNAs were then stimulated with increasing concentrations (0.1µM/0.3µM/1µM/3µM/10µM/30µM/60µM/100µM) of patchouli oil (Takasago, molecular weight: 500) and a patchouli oil fraction enriched in patchouli alcohol, denoted R2335, commercially available and provided by Robertet under the reference "Patchouli Heart" or "Patchouli Coeur".

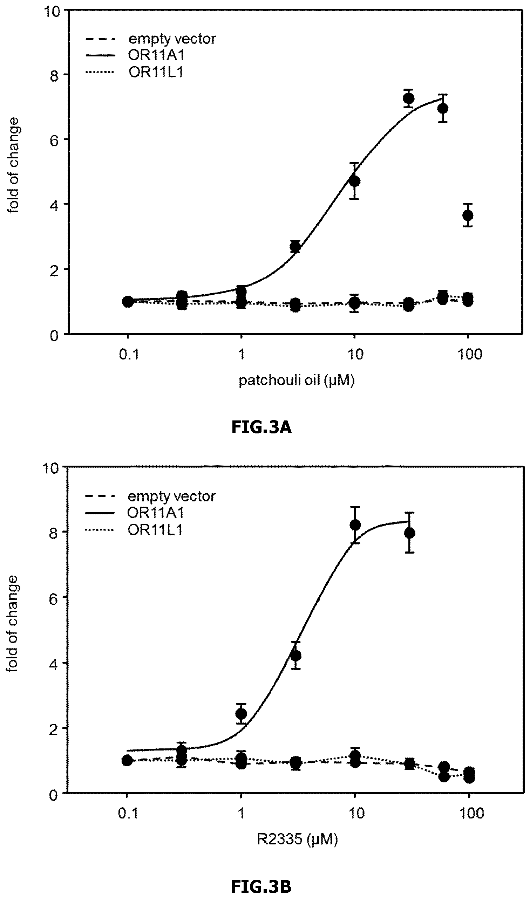

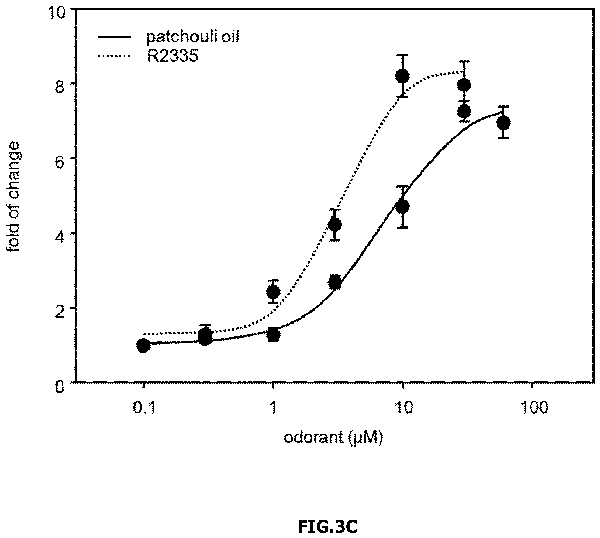

Cells transfected with OR11A1 responded to patchouli oil and R2335 in a concentration-dependent manner (figure 3C). Interestingly, neither compound was able to activate OR11L1, a human OR that belongs to the same subfamily of 8 receptors (figures 3A and 3B). R2335 had a slightly higher potency on OR11A1 (EC50 3.3 ± 0.8 mM) than patchouli oil (EC50 7.6 ± 0.6 mM). Notably, the application of both odorants in concentrations above 30 µM led to a strong signal reduction, presumably due to unspecific, toxic interactions of both compounds with the cells. Altogether, these results show that OR11A1 is specifically responsive to patchouli odorants.

### Example 3: Response of OR11A1 related receptors towards different odorants

To investigate the response of OR11A1 related receptors towards different odorants (patchouli alcohol (molecular weight (MW) 222) and patcho 315 (MW 220), the structure of the latter being shown below), the cells were transiently transfected with plasmid DNA for seven OR11A1 related receptors belonging to a small subset of structurally related receptors (see figure 2) and a control plasmid that served as a negative control.

The different odorants tested on OR11A1 and OR11L1 related receptors are as mentioned in Table 3:

**Table 3**

| **Compound** | **Concentrations used (µM)** |
|---|---|
| Patchouli alcohol | 10/30/100 |
| Patcho 315* | 10/30/100 |

| | |
|---|---|
| * 6a,7,7-trimethyldecahydro-1,4-methanocyclopropa[de]naphtalen-1-ol (Chemical Formula: C₁₄H₂₂O; exact mass: 206.17), disclosed in Japanese patent applications n° 2012-103867 and n° 2012-103868. | |

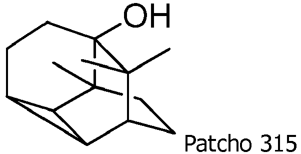

24 hours after transfection, the cells were stimulated with three different concentrations of the odorants as mentioned above.

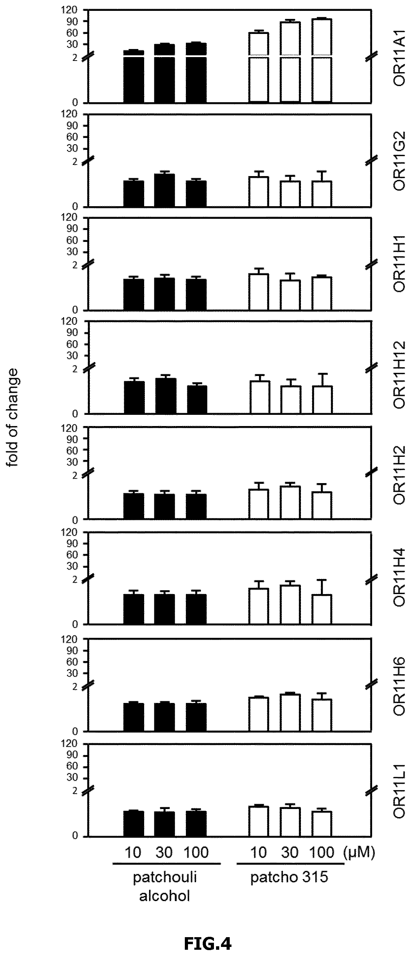

The results are shown in Figure 4. As observed in previous experiments, patcho 315 and patchouli alcohol strongly activated OR11A1 but were unable to elicit a detectable response in cells transfected with OR11L1. In addition, none of the seven other OR11A1-related olfactory receptors responded to the tested odorants (figure 4, fold of change around 1).

A possible explanation for this could be the relatively low amino acid identity of the seven OR11A1 related olfactory receptors compared to OR11A1, which is not higher than 46%.

### Example 4: Response of OR11A1 towards different odorants

To investigate the response of OR11A1 receptor towards different odorants (patchouli alcohol, patcho 315, terranol (CAS N° 57566-26-4, MW 222), andrane (CAS N° 13567-39-0, MW 220), pogostone (CAS N° 23800-56-8, MW 224), norpatchouli alcohol, MW 206), HEKOlf cells were transiently transfected with plasmid DNA for OR11A1 receptor as described above.

The different odorants tested on the OR11A1 receptor are as mentioned in Table 4:

**Table 4**

| **Compound** | **Concentrations used (µM)** |
|---|---|
| Patchouli alcohol | 0.03/0.1/0.3/1/3/6/10/30/100 |
| Patcho 315 | 0.03/0.1/0.3/1/3/6/10/30/100 |
| Terranol | 3/10/30 |
| Andrane | 3/10/30 |
| Pogostone | 0.03/0.1/0.3/1/3/6/10/30/100 |
| Norpatchouli alcohol | 0.03/0.1/0.3/1/3/6/10/30/100 |

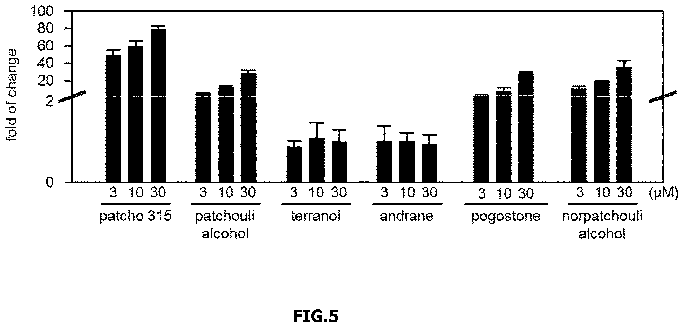

The results obtained with testing concentrations of 3, 10 and 30 µM are shown in Figure 5.

As observed in previous experiments, patcho 315 strongly activated OR11A1. In addition, patchouli alcohol also activated OR11A1. This confirms again the activity of these two patchouli odorants on OR11A1. From the four newly tested odorants only pogostone and norpatchouli alcohol were able to elicit activation in OR11A1 transfected cells. Terranol and andrane did not activate OR11A1 up to 100µM. Cells that were transfected with the control plasmid did not respond to any of the odorants tested (not shown).

Four different patchouli related odorants were further tested as described above for their ability to activate OR11A1, the receptor identified to specifically respond to patchouli odorants.

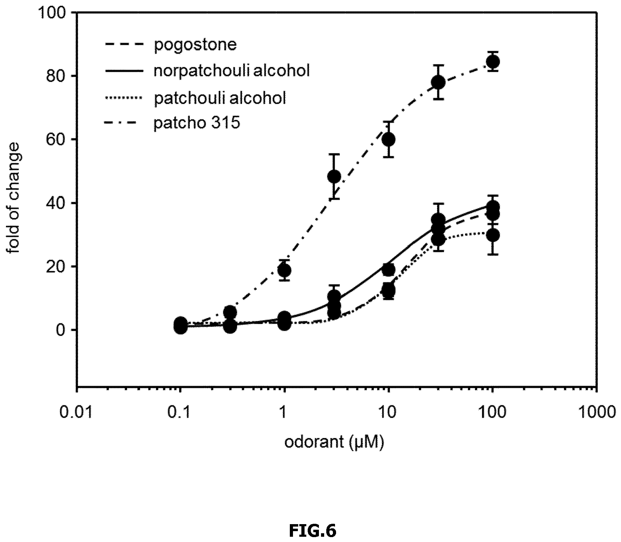

Results obtained with larger concentrations of patcho 315, patchouli alcohol, norpatchouli alcohol and pogostone starting from 0.1 to 100 µM are shown in Figure 6.

The results show that OR11A1 receptor transfected cells responded to the different odorants in a concentration-dependent manner. Potency and efficacy on OR11A1 was highest for Patcho 315. The activation of OR11A1 by patchouli alcohol, norpatchouli alcohol and pogostone was comparable, indicated by only minor differences in EC₅₀ values (table 5).

**Table 5**

| **Compound** | **EC50 (µM)** |
|---|---|
| Patcho 315 | 2.9 ± 0.9 |
| Patchouli alcohol | 12.1 ± 1.7 |
| Pogostone | 14.8 ± 3.3 |
| Norpatchouli alcohol | 10.7 ± 2.6 |

### Example 5: "long-lasting" analysis of patchouli alcohol

50 µl of a 1 % solution of patchouli alcohol in ethanol was added by pipette to the tip to a series of different scent strips (SARL H. GRANGER-VEYRON, Privas, France). The strips were allowed to dry under ambient laboratory conditions.

After different times (t0, t0+1hour, t0+2hours, t0+4hours, t0+8hours) the tip of a strip where the 50 µl of the solution of the tested material had been applied was cut and put in a 30 ml bottle and extracted using 3 ml of ethanol, containing 50 µl of methyl decanoate as internal standard (10000 ppm solution in cyclohexane), added to each bottle. Care was taken to ensure the entire area around the point of application was included in the area that was cut and extracted. The content of the bottles was mixed for 30 min on a roller bed, and 2 ml vials were then filled with each sample.

Samples were triplicated, i.e. 3 scent strip tips were analysed for each time point.

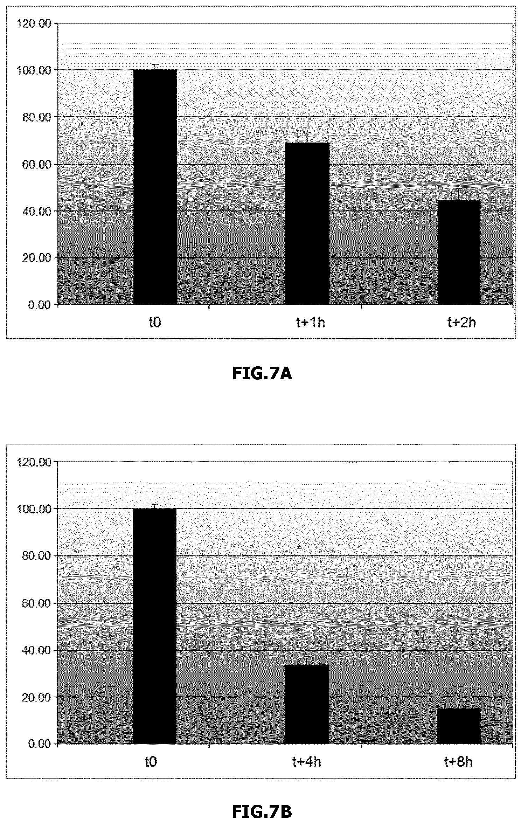

In a first series of experiments, samples taken at t0, t0+1hour and t0+2hours were analyzed by GC-MS on a non-polar gas chromatography column (column BC-WAX (50m * 0.25mm * 0.15µm) which is a GC Sciences column supplied by Interchim, Montluçon, France). The results are shown in Tables 6-8 and in Figure 7A.

In a second series of experiments, samples taken at t0, t0+4hours and t0+8hours were analyzed by GC-MS on a non-polar gas chromatography column (HP-5MS UI (30m * 0.25mm * 0.25µm) which is an Agilent column supplied by Chromoptic, Courtaboeuf, France). The results are shown in Tables 9-11 and in Figure 7B.

In Tables 6-11, PA stands for patchouli alcohol and IS stands for the internal standard, i.e. methyl decanoate. When plotting the residual concentration of patchouli on the scent strip as a function of time, the mean ratio at t0 was normalized to 100 and then t0+1h, t0+2h, t0+4h and t0+8h were calculated; t0 being defined as 2 minutes after dosing the 50 µl of the solution of the tested material, which was applied towards the tip of the scent strip.

These results show that PA retains at least 10% of its initial concentration after 1 hour when applied to a specified substrate.

**Table 6: t0 (PA)**

| | sample 1 | sample 2 | sample 3 | mean value | std dev % |
|---|---|---|---|---|---|
| PA | 2.98E+06 | 2.87E+06 | 2.94E+06 | 2.93E+06 | 1.84 |
| IS | 3.16E+06 | 2.90E+06 | 3.03E+06 | 3.03E+06 | 4.28 |
| Ratio PA/IS | 9.42E-01 | 9.89E-01 | 9.69E-01 | 9.67E-01 | 2.47 |

**Table 7: t0+1h (PA)**

| | sample 1 | sample 2 | sample 3 | mean value | std dev % |
|---|---|---|---|---|---|
| PA | 2.10E+06 | 2.12E+06 | 2.05E+06 | 2.09E+06 | 1.66 |
| IS | 3.23E+06 | 2.96E+06 | 3.19E+06 | 3.13E+06 | 4.76 |
| Ratio PA/IS | 6.50E-01 | 7.16E-01 | 6.43E-01 | 6.70E-01 | 6.07 |

**Table 8: t0+2h (PA)**

| | sample 1 | sample 2 | sample 3 | mean value | std dev % |
|---|---|---|---|---|---|
| PA | 1.38E+06 | 1.32E+06 | 1.35E+06 | 1.35E+06 | 2.38 |
| IS | 3.70E+06 | 2.91E+06 | 2.93E+06 | 3.18E+06 | 14.04 |
| Ratio PA/IS | 3.74E-01 | 4.52E-01 | 4.62E-01 | 4.29E-01 | 11.25 |

**Table 9: t0 (PA)**

| | sample 1 | sample 2 | sample 3 | mean value | std dev % |
|---|---|---|---|---|---|
| PA | 9.37E+06 | 9.23E+06 | 8.94E+06 | 9.18E+06 | 2.41 |
| IS | 7.51E+06 | 7.57E+06 | 7.09E+06 | 7.39E+06 | 3.54 |
| Ratio PA/IS | 1.25E+00 | 1.22E+00 | 1.26E+00 | 1.24E+00 | 1.71 |

**Table 10: t0+4h (PA)**

| | sample 1 | sample 2 | sample 3 | mean value | std dev % |
|---|---|---|---|---|---|
| PA | 3.83E+06 | 2.94E+06 | 3.00E+06 | 3.26E+06 | 15.28 |
| IS | 8.21E+06 | 7.57E+06 | 7.71E+06 | 7.83E+06 | 4.33 |
| Ratio PA/IS | 4.67E-01 | 3.89E-01 | 3.89E-01 | 4.15E-01 | 10.80 |

**Table 11: t0+8h (PA)**

| | sample 1 | sample 2 | sample 3 | mean value | std dev % |
|---|---|---|---|---|---|
| PA | 2.02E+06 | 1.37E+06 | 1.35E+06 | 1.58E+06 | 24.26 |
| IS | 9.36E+06 | 7.73E+06 | 8.17E+06 | 8.42E+06 | 10.02 |
| Ratio PA/IS | 2.16E-01 | 1.77E-01 | 1.65E-01 | 1.86E-01 | 14.37 |

### Example 6: "long-lasting" analysis of pogostone

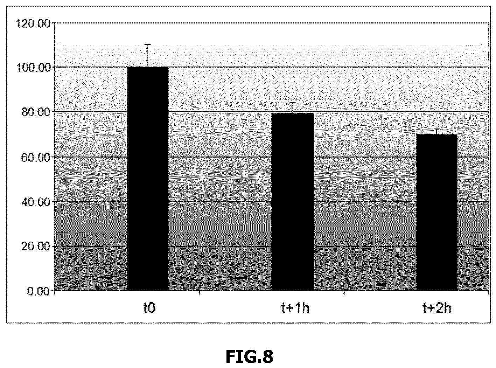

A "long-lasting" analysis of pogostone was performed as described in Example 5 with samples taken at t0, t0+1hour and t0+2hours. The results are shown in Tables 12-14 and in Figure 8.

In Tables 12-14 herebelow, POG stands for pogostone and IS stands for the internal standard, i.e. methyl decanoate. The mean ratio at t0 was normalized as described in Example 5.

These results show that POG retains at least 10% of its initial concentration after 1 hour.

**Table 12: t0 (POG)**

| | sample 1 | sample 2 | sample 3 | mean value | std dev % |
|---|---|---|---|---|---|
| POG | 1.17E+06 | 1.06E+06 | 1.09E+06 | 1.11E+06 | 4.71 |
| IS | 4.00E+06 | 4.34E+06 | 4.42E+06 | 4.25E+06 | 5.27 |
| Ratio POG/IS | 2.92E-01 | 2.45E-01 | 2.48E-01 | 2.61E-01 | 10.01 |

**Table 13: t0+1h (POG)**

| | sample 1 | sample 2 | sample 3 | mean value | std dev % |
|---|---|---|---|---|---|
| POG | 1.05E+06 | 9.11E+05 | 9.09E+05 | 9.55E+05 | 8.19 |
| IS | 4.71E+06 | 4.60E+06 | 4.47E+06 | 4.59E+06 | 2.58 |
| Ratio POG/IS | 2.22E-01 | 1.98E-01 | 2.03E-01 | 2.08E-01 | 6.05 |

**Table 14: t0+2h (POG)**

| | sample 1 | sample 2 | sample 3 | mean value | std dev % |
|---|---|---|---|---|---|
| POG | 8.11E+05 | 8.75E+05 | 8.15E+05 | 8.34E+05 | 4.32 |
| IS | 4.64E+06 | 4.69E+06 | 4.39E+06 | 4.57E+06 | 3.43 |
| Ratio POG/IS | 1.75E-01 | 1.87E-01 | 1.85E-01 | 1.82E-01 | 3.59 |

### References

- Bufe B, Hofmann T, Krautwurst D, Raguse JD, Meyerhof W.
   The human TAS2R16 receptor mediates bitter taste in response to beta-glucopyranosides. Nat Genet. 2002 Nov;32(3):397-401.
- Von Dannecker LE, Mercadante AF, Malnic B.
   Ric-8B, an olfactory putative GTP exchange factor, amplifies signal transduction through the olfactory-specific G-protein Galphaolf.
   J Neurosci. 2005 Apr 13;25(15):3793-800.
- Von Dannecker LE, Mercadante AF, Malnic B.
   Ric-8B promotes functional expression of odorant receptors.
   Proc Natl Acad Sci USA. 2006 Jun 13;103(24):9310-4.
- Malnic B, Hirono J, Sato T, Buck LB.
   Combinatorial receptor codes for odors.
   Cell. 1999 Mar 5;96(5):713-23.
- Glusman G, Bahar A, Sharon D, Pilpel Y, White J, Lancet D.
   The olfactory receptor gene superfamily: data mining, classification, and nomenclature. Mamm Genome. 2000 Nov;11(11): 1016-23.
- Keller A, Zhuang H, Chi Q, Vosshall LB, Matsunami H.
   Genetic variation in a human odorant receptor alters odour perception.
   Nature. 2007 Sep 27;449(7161):468-72.
- Shirokova E, Schmiedeberg K, Bedner P, Niessen H, Willecke K, Raguse JD, Meyerhof W, Krautwurst D.
   Identification of specific ligands for orphan olfactory receptors.
   J Biol Chem. 2005 Mar 25;280(12):11807-15.

## Claims

1. A method for identifying a candidate patchouli odorant material, comprising the steps of:
1) contacting a sample expressing the OR11A1 receptor with a test compound, and
2) measuring the activity of the OR11A1 receptor by a method comprising:
i) measuring the response of the OR11A1 receptor to the test compound and the response of the OR11A1 receptor to the assay buffer (baseline), and calculating a first fold of change (FOC1: response OR11A1 to test compound/response OR11A1 to baseline);
ii) measuring the response of a control sample which does not express OR11A1 receptor to the test compound and the response of the control sample which does not express the OR11A1 receptor to the assay buffer (baseline), and calculating a second fold of change (FOC2: response control sample to test compound/response control sample to baseline);
wherein the test compound is identified as a candidate patchouli odorant material if the ratio of FOC1 to FOC2 is higher than 1.

2. The method of claim 1, wherein said sample expresses a functional OR11A1 receptor.

3. The method of any one of claims 1 to 2, wherein said sample comprises a cell.

4. The method of claim 3, wherein said cell is a mammalian cell.

5. The method of any one of claims 3 or 4, wherein the sample is a cell line selected from the group consisting of HEK293T, CHO (Chinese hamster ovary), HeLa and membranes preparations of said cells.

6. The method of any one of claims 1 to 5, wherein the OR11A1 receptor is human OR11A1 receptor encoded (i) by the nucleotide sequence of sequence SEQ ID NO:1, or (ii)by a nucleotide sequence encoding the polypeptide of SEQ ID NO:2, or (iii) by a nucleotide sequence encoding a polypeptide having an amino acid sequence identity of at least 85 %, preferably at least 90 % and more preferably at least 95 %, with SEQ ID NO:2.

7. The method of any one of claims 1 to 6, wherein said test compound has a molecular weight of greater than 183, preferably equal to or greater than 200.

8. The method of any one of claims 1 to 7, wherein said test compound has a molecular weight of 300 or less, preferably of 275 or less.

9. The method of any one of claims 1 to 8, wherein FOC1 is of at least 1.5, preferably of at least 1.8 and more preferably of at least 2.

10. The method of any one of claims 1 to 9, wherein said activation of OR11A1 receptor is detected by using a reporting system leading to a readable or measureable signal if the OR11A1 receptor is activated such as, but not limited to calcium imaging with fluorescent dyes, photoproteins such as GFP, aequorin, enzymatic reporter system such as luciferase, chloramphenicol acetyltransferase, β-galactosidase, alkaline phosphatase, horse radish peroxidase, GTPgammaS binding test, measurement of changes in cAMP levels, and the like, preferably by using a reporting system comprising a gene whose transcription is driven by a response element whose activity is an indication of OR11A1 receptor activation, preferably driven by cAMP response element which is stimulated by adenylate cyclase which is activated by the stimulatory G-protein (Gas), such as Gαolf G-protein.

11. A method for identifying a patchouli odorant material, comprising the steps of:
a) identifying a candidate patchouli odorant material by the method of any one of claims 1 to 10, and
b1) subjecting the identified candidate to a long-lasting test, wherein the candidate passes the test if it retains at least 10% of its initial concentration after 1 hour; and/or b2) subjecting the identified candidate to an olfactive test in which a sample containing patchouli oil is compared with a sample containing patchouli oil and the identified candidate, wherein the candidate passes the test if there is no perceptible sensory difference between the two samples.

12. The method according to claim 11, wherein the long-lasting test comprises the steps of:
- applying 50 µl of a 1 % solution in alcohol of the identified candidate, on a scent strip;
- allowing the scent strip to dry under ambient conditions;
- extracting at least the part of the scent strip, including the area where the solution of the identified candidate was applied with 3 ml of alcohol containing an internal standard;
- analysing the extract by gas chromatography;
- normalizing the result for the principal molecular ion of the identified candidate using the value for the internal standard.

13. The method according to claim 11, wherein the olfactive test is carried out following the protocol defined in standard ASTM E 1885 - 97.

14. The method according to any one of claims 10 to 13, comprising steps a), b1) and b2), wherein the steps are carried out in the following order: a), b1), b2).

15. The method according to any one of claims 10 to 13, comprising steps a), b1) and b2), wherein the steps are carried out in the following order: a), b2), b1).

16. Use of a sample expressing the OR11A1 receptor for identifying a candidate patchouli odorant material upon enhancement of the activity of said receptor by said material.

17. The use of claim 16, wherein the OR11A1 receptor is human OR11A1 receptor encoded by the nucleotide sequence of sequence SEQ ID NO:1, or by a nucleotide sequence encoding the polypeptide of SEQ ID NO:2.

## Patentansprüche

1. Verfahren zum Identifizieren eines Patschuligeruchsstoffmaterialkandidaten, umfassend die Schritte:
1) Inkontaktbringen einer Probe, die den OR11A1-Rezeptor exprimiert, mit einer Testverbindung und
2) Messen der Aktivität des OR11A1-Rezeptors mittels eines Verfahrens, das umfasst:
i) Messen der Antwort des OR11A1-Rezeptors auf die Testverbindung und der Antwort des OR11A1-Rezeptors auf den Testpuffer (Ausgangswert) und Berechnen eines ersten Unterschiedskoeffizient (FOC1: Antwort OR11A1 auf Testverbindung / Antwort OR11A1 auf Ausgangswert),
ii) Messen der Antwort eine Kontrollprobe, die keinen OR11A1-Rezeptor exprimiert, auf die Testverbindung und der Antwort der Kontrollprobe, die keinen OR11A1-Rezeptor exprimiert, auf den Testpuffer (Ausgangswert) und Berechnen eines zweiten Unterschiedskoeffizient (FOC2: Antwort Kontrollprobe auf Testverbindung / Antwort Kontrollprobe auf Ausgangswert),
wobei die Testverbindung als ein Patschuligeruchsstoffmaterialkandidat identifiziert wird, wenn das Verhältnis von FOC1 zu FOC2 größer als 1 ist.

2. Verfahren nach Anspruch 1, wobei die Probe einen funktionellen OR11A1-Rezeptor exprimiert.

3. Verfahren nach einem der Ansprüche 1 bis 2, wobei die Probe eine Zelle umfasst.

4. Verfahren nach Anspruch 3, wobei die Zelle eine Säugetierzelle ist.

5. Verfahren nach einem der Ansprüche 3 oder 4 ist, wobei die Probe eine Zelllinie ist, die aus der aus HEK293T, CHO (Ovarzellen des chinesischen Hamsters), HeLa und Membranzubereitungen der Zellen bestehenden Gruppe ausgewählt ist.

6. Verfahren nach einem der Ansprüche 1 bis 5, wobei der OR11A1-Rezeptor ein humaner OR11A1-Rezeptor ist, der (i) durch die Nukleotidsequenz mit der Sequenz SEQ ID NR. 1 oder (ii) durch eine Nukleotidsequenz, die das Polypeptid SEQ ID NR. 2 codiert, oder (iii) durch eine Nukleotidsequenz, die ein Polypeptid codiert, das eine Aminosäuresequenz aufweist, die wenigstens 85 %, vorzugsweise wenigstens 90 % und weiter bevorzugt wenigstens 95 % Identität mit SEQ ID NR. 2 aufweist, codiert wird.

7. Verfahren nach einem der Ansprüche 1 bis 6, wobei die Testverbindung ein Molekulargewicht von mehr als 183, vorzugsweise gleich oder mehr als 200 aufweist.

8. Verfahren nach einem der Ansprüche 1 bis 7, wobei die Testverbindung ein Molekulargewicht von 300 oder weniger, vorzugsweise 275 oder weniger aufweist.

9. Verfahren nach einem der Ansprüche 1 bis 8, wobei FOC1 wenigstens 1,5, vorzugsweise wenigstens 1,8 und weiter bevorzugt wenigstens 2 beträgt.

10. Verfahren nach einem der Ansprüche 1 bis 9, wobei die Aktivierung des OR11A1-Rezeptors detektiert wird, in dem ein Berichtssystem verwendet wird, das zu einem lesbaren oder messbaren Signal führt, wenn der OR11A1-Rezeptor aktiviert wird, wie zum Beispiel, ohne darauf beschränkt zu sein, Calcium-Imaging mit Fluoreszenzfarbstoffen, Fotoproteine wie GFP, Aequorin, enzymatische Berichtssysteme wie Luciferase, Chloramphenicolacetyltransferase, β-Galactosidase, alkalische Phosphatase, Meerrettich-Peroxidase, GTPgammaS-Bindungstest, Messung von cAMP-Niveau-Änderungen und dergleichen, vorzugsweise in dem ein Berichtssystem verwendet wird, das ein Gen umfasst, dessen Transkription durch ein Antwortelement angetrieben wird, dessen Aktivität ein Indikator der OR11A1-Rezeptor-Aktivierung ist, vorzugsweise durch ein cAMP-Antwortelement angetrieben wird, das durch Adenylatcyclase stimuliert wird, die durch das stimulierende G-Protein (GαS) wie das Gαolf-G-Protein, aktiviert wird.

11. Verfahren zum Identifizieren eines Patschuligeruchsstoffmaterials, umfassend die Schritte:
a) Identifizieren eines Patschuligeruchsstoffmaterialkandidaten mittels des Verfahrens nach einem der Ansprüche 1 bis 10 und
b1) Unterziehen des Kandidaten einem Langzeittest, wobei der Kandidat den Test besteht, wenn er wenigstens 10 % seiner ursprünglichen Konzentration nach 1 Stunde beibehält, und/oder
b2) Unterziehen des identifizierten Kandidaten einem olfaktorischen Test, in dem eine Probe, die Patschuliöl enthält, mit einer Probe, die Patschuliöl und den identifizierten Kandidaten enthält, verglichen wird, wobei der Kandidat den Test besteht, wenn zwischen den beiden Proben kein wahrnehmbarer Geruchsunterschied besteht.

12. Verfahren nach Anspruch 11, wobei der Langzeittest die Schritte umfasst:
- Auftragen von 50 µl einer 1 %-igen Lösung des identifizierten Kandidaten in Alkohol auf einen Duftstreifen,
- Trockenlassen des Duftstreifens unter Umgebungsbedingungen,
- Extrahieren wenigstens des Teils des Duftstreifens, der den Bereich umfasst, auf dem die Lösung des identifizierten Kandidaten aufgetragen wurde, mit 3 ml Alkohol, der einen internen Standard enthält,
- Analysieren des Extraktes mittels Gaschromatographie,
- Normalisieren des Ergebnisses auf das hauptsächlich vorhandene Molekülion des identifizierten Kandidaten unter Verwendung des Wertes für den internen Standard.

13. Verfahren nach Anspruch 11, wobei der olfaktorische Test durchgeführt wird, in dem das im Standard ASTM E 1885-97 definierte Protokoll befolgt wird.

14. Verfahren nach einem der Ansprüche 10 bis 13, umfassend die Schritte a), b1) und b2), wobei die Schritte in der folgenden Reihenfolge durchgeführt werden: a), b1), b2).

15. Verfahren nach einem der Ansprüche 10 bis 13, umfassend die Schritte a), b1) und b2), wobei die Schritte in der folgenden Reihenfolge durchgeführt werden: a), b2), b1).

16. Verwendung einer Probe, die den OR11A1-Rezeptor exprimiert, zum Identifizieren eines Patschuligeruchsstoffmaterialkandidaten durch Verbesserung der Aktivität des Rezeptors durch das Material.

17. Verwendung nach Anspruch 16, wobei der OR11A1-Rezeptor ein humaner OR11A1-Rezeptor ist, der durch die Nukleotidsequenz mit der Sequenz SEQ ID NR. 1 oder durch eine Nukleotidsequenz, die das Polypeptid SEQ ID NR. 2 codiert, codiert wird.

## Revendications

1. Méthode pour identifier une matière odorante de patchouli candidate, qui comprend les étapes de :
1) mise en contact d'un échantillon exprimant le récepteur OR11A1 avec un composé test, et
2) mesure de l'activité du récepteur OR11A1 par une méthode comprenant :
i) mesure de la réponse du récepteur OR11A1 au composé test et mesure de la réponse du récepteur OR11A1 au tampon de dosage (niveau de référence), et calcul d'un premier coefficient de variation (FOC1 : réponse OR11A1 au composé test/réponse OR11A1 au niveau de référence);
ii) mesure de la réponse d'un échantillon témoin qui n'exprime pas le récepteur OR11A1 au composé test et de la réponse de l'échantillon témoin qui n'exprime pas le récepteur OR11A1 au tampon de dosage (niveau de référence), et calcul d'un second coefficient de variation (FOC2 : réponse de l'échantillon témoin au composé test/réponse de l'échantillon témoin au niveau de référence);
dans laquelle le composé test est identifié comme une matière odorante de patchouli candidate si le rapport de FOC1 à FOC2 est supérieur à 1.

2. Méthode selon la revendication 1, dans laquelle ledit échantillon exprime un récepteur OR11A1 fonctionnel.

3. Méthode selon la revendication 1 ou la revendication 2, dans laquelle ledit échantillon comprend une cellule.

4. Méthode selon la revendication 3, dans laquelle ladite cellule est une cellule de mammifère.

5. Méthode selon l'une quelconque des revendications 3 et 4, dans laquelle l'échantillon est une lignée cellulaire choisie dans le groupe constitué de HEK293T, CHO (ovaire de hamster chinois), HeLa et les préparations membranaires de ces cellules.

6. Méthode selon l'une quelconque des revendications 1 à 5, dans laquelle le récepteur OR11A1 est le récepteur OR11A1 humain encodé (i) par la séquence nucléotidique SEQ ID NO:1, ou (ii) par une séquence nucléotidique qui code le polypeptide of SEQ ID NO:2, ou (iii) par une séquence nucléotidique qui code un polypeptide ayant une identité de séquence d'acides aminés d'au moins 85%, de préférence d'au moins 90%, et de préférence encore d'au moins 95%, avec SEQ ID NO:2.

7. Méthode selon l'une quelconque des revendications 1 à 6, dans laquelle ledit composé test a une masse moléculaire supérieure à 183, de préférence supérieure ou égale à 200.

8. Méthode selon l'une quelconque des revendications 1 à 7, dans laquelle ledit composé test a une masse moléculaire inférieure ou égale à 300, de préférence inférieure ou égale à 275.

9. Méthode selon l'une quelconque des revendications 1 à 8, dans laquelle FOC1 est au moins égal à 1,5, de préférence au moins égal à 1,8, de préférence encore au moins égal à 2.

10. Méthode selon l'une quelconque des revendications 1 à 8, dans laquelle l'activation du récepteur OR11A1 est détectée en utilisant un système rapporteur conduisant à un signal mesurable ou lisible si le récepteur OR11A1 est activé, tel que mais de manière non limitative : imagerie calcique avec des colorants fluorescents, photoprotéines telles que GFP, aequorine, système rapporteur enzymatique tel que la luciférase, chloramphénicol acétyltransférase, β-galactosidase, phosphatase alcaline, peroxydase de raifort, test de liaison au GTPgammaS, mesure de variation du niveau d'AMPc, et analogues, de préférence en utilisant un système rapporteur comprenant un gène dont la transcription est assurée par un élément de réponse dont l'activité est un indicateur de l'activation du récepteur OR11A1, de préférence assurée par un élément de réponse AMPc qui est stimulé par l'adénylate cyclase qui est activée par la protéine G stimulatrice (Gαs), telle que la protéine G Gαolf.

11. Méthode pour identifier une matière odorante de patchouli, qui comprend les étapes de :
a) identification d'une matière odorante de patchouli candidate par la méthode selon l'une quelconque des revendications 1 à 10, et
b1) soumission du candidat identifié à un test longue durée, le candidat réussissant le test s'il conserve au moins 10% de sa concentration initiale après 1 heure; et/ou
b2) soumission du candidat identifié à un test olfactif dans lequel un échantillon contenant de l'huile de patchouli est comparé à un échantillon contenant de l'huile de patchouli et le candidat identifié, le candidat réussissant le test s'il n'y a pas de différence sensorielle perceptible entre les deux échantillons.

12. Méthode selon la revendication 11, dans laquelle le test longue durée comprend les étapes de :
- appliquer 50 µl d'une solution à 1 % dans l'alcool du candidat identifié, sur une bandelette de test ;
- laisser la bandelette de test sécher à l'air ambiant ;
- extraire au moins la partie de la bandelette de test incluant la zone où la solution du candidat identifié a été appliquée, avec 3 mL d'alcool contenant une référence interne ;
- analyser l'extrait par chromatographie gazeuse ;
- normaliser le résultat pour l'ion moléculaire principal du candidat identifié en utilisant la valeur de la référence interne.

13. Méthode selon la revendication 11, dans laquelle le test olfactif est effectué en suivant le protocole défini dans la norme ASTM E 1885 - 97.

14. Méthode selon l'une quelconque des revendications 10 à 13, comprenant les étapes a), b1) et b2), dans laquelle les étapes sont réalisées dans l'ordre suivant : a), b1), b2).

15. Méthode selon l'une quelconque des revendications 10 à 13, comprenant les étapes a), b1) et b2), dans laquelle les étapes sont réalisées dans l'ordre suivant : a), b2), b1).

16. Utilisation d'un échantillon exprimant le récepteur OR11A1 pour identifier une matière odorante de patchouli candidate par xx de l'activité dudit récepteur par ladite matière.

17. Utilisation selon la revendication 16, dans laquelle le récepteur OR11A1 est le récepteur OR11A1 humain encodé par la séquence nucléotidique de séquence SEQ ID NO:1, ou par une séquence nucléotidique qui code polypeptide de SEQ ID NO:2.
